# EUROPEAN PATENT SPECIFICATION

(11) **EP 2 651 362 B1**
(45) Date of publication and mention of the grant of the patent: **10.08.2016**
(21) Application number: 10860840.7
(22) Date of filing: 13.12.2010
(51) Int. Cl.: A61F 13/56, C09D 5/28, C09D 7/12, C09D 11/02, C09J 7/02, A61L 15/50

(54) **FASTENING MEANS FOR AN ABSORBENT PRODUCT**
BEFESTIGUNGSMITTEL FÜR EIN SAUGFÄHIGES PRODUKT
MOYENS DE FIXATION POUR UN PRODUIT ABSORBANT

(43) Date of publication of application: 23.10.2013
(73) Proprietor: SCA Hygiene Products AB, 405 03 Göteborg (SE)
(72) Inventor: DREVIK, Solgun, S-435 35 Mölnlycke (SE); BURVALL, Angelica, S-517 37 Bollebygd (SE)
(74) Representative: Zacco Sweden AB
(86) International application number: PCT/SE2010/051373
(87) International publication number: WO 2012/082027

(56) References cited:
- EP-A1- 0 471 385
- EP-A2- 0 229 639
- EP-A2- 0 229 639
- WO-A1-91/13752
- WO-A1-91/13752
- WO-A1-99/20217
- WO-A2-02/08067
- FR-A1- 2 366 009
- US-A- 4 337 772
- US-A- 4 376 440
- US-A- 4 834 739
- US-A- 4 850 991
- US-A- 4 959 265
- US-A- 5 514 122

## Description

### TECHNICAL FIELD

The present invention pertains to a method of applying a fastening means to the backsheet of an absorbent product, to an absorbent product comprising such fastening means, and the use of an expandable ink in combination with an adhesive layer as a fastening means.

### BACKGROUND ART

Absorbent products such as panty liners, sanitary napkins and incontinence pads are typically held in place by pressure sensitive adhesive, e.g. in the form of a double sided tape or a hot melt adhesive applied on the garment facing side of the product. The adhesive holds the absorbent product in place by adhering it to the crotch of the user's underwear. The pressure sensitive adhesive is covered with release paper that protects the adhesive from unintended adhesion during manufacture, packaging and storage. The release paper must be removed by the user before application of the product to the underwear, and thus creates waste. US6004308A discloses an adhesive attachment system for sanitary napkins, including non-tacky particles adhered to a pressure sensitive adhesive. EP0623332A1 discloses a method of protecting an adhesive by placing it in depressions on the backsheet of an absorbent article. US 5514122 discloses a feminine hygiene pad provided with a linerless, microsphere pressure sensitive adhesive attachment region. These methods make the use of release paper unnecessary, but involve relatively complicated manufacture of the products.

### SUMMARY OF THE INVENTION

The present invention aims at providing an improved method of applying a fastening means to absorbent products, whereby the need of a release paper during manufacture, packaging and storage is avoided. By means of the invention a versatile and flexible fastening means is achieved on the absorbent product.

In the method of the invention a fastening means is applied to a backsheet of an absorbent product, said backsheet facing away from the user during use, and being opposite to a body facing side of the absorbent product. The method comprises the steps of applying an adhesive layer on the backsheet of the absorbent product, and applying an expandable ink covering 5-95% of the backsheet.

The method may comprise a step of drying the applied ink, and preferably comprises a step of activating of the expandable ink, which may be performed by exposing the expandable ink to heat. The adhesive may be a pressure sensitive hot melt adhesive, and if so may be applied at a temperature of 100-180 °C, and the expandable ink may be applied while the pressure sensitive hot melt adhesive is still at temperature above 80°C, thus achieving activation by means of the heat of the adhesive. Further, the expandable ink may be activated by means of a heated embossing roll, infra-red radiation, hot air, or microwaves. If desired, the expandable ink may instead be activated by means of UV radiation. The expandable ink may be activated in selected areas only, and/or may be applied in selected areas. The expandable ink preferably comprises a polymer, a solvent and a blowing agent or expandable microspheres.

An absorbent product according to the invention comprises a body facing side, and a backsheet facing away from the user during use, and being opposite to the body facing side of the absorbent product. A fastening means is applied on the backsheet, and comprises an adhesive layer and an expandable ink. Advantageously, the expandable ink has been activated so as to form three-dimensionally extending protrusions, to make the absorbent product ready for use. The three-dimensionally extending protrusions preferably have a height of 0.1-8 mm, most preferably 0.1-3 mm. Preferably 5-95% of the backsheet is covered by the expandable ink, more preferably 10-60 %.

The invention also relates to the use of an expandable ink in combination with an adhesive layer as a fastening means for an absorbent product.

### BRIEF DESCRIPTION OF THE DRAWINGS

- Figure 1: shows a suitable pattern which can be used in the present invention;
- Figure 2: shows an alternative suitable pattern which can be used in the present invention.

### DETAILED DESCRIPTION

The present invention relates to a method of applying a fastening means to an absorbent product, and to an absorbent product including such fastening means, and to the use of expandable ink in a fastening means for an absorbent product. The fastening means, when ready for use, comprises adhesive areas and protruding non-adhesive areas. The protruding non-adhesive areas act as distances and will protect the adhesive from unintentional contact, and will prevent the adhesive areas from coming into contact with any surface unless it is pressed against the surface. Thus, the fastening means need not be protected by a release sheet during manufacture, packaging or storage. When attaching the absorbent product to a surface, e.g. a textile surface, the protruding areas acting as distances and will initially prevent contact between the adhesive and the surface. Due to friction between the protruding areas and the surface the absorbent product stays in place. When pressure is exerted on the absorbent product by a user in the direction towards the surface, the protruding areas will be compressed and/or the material of the surface will be draped around the protruding areas, and the adhesive will eventually come into contact with the surface of e.g. panty or mattress, thus achieving a more secure fastening of the absorbent product. Further, due to friction, obtained by means of the protruding areas, keeping the product in place before the adhesive has come into contact with the surface, the position of the absorbent product can easily be adjusted, while being retained safely enough to the surface.

In the method of the present invention a fastening means is applied to the backsheet of an absorbent product. The absorbent product may for example be a sanitary napkin, a panty liner, an incontinence pad, a diaper, or any other absorbent product that is adapted to be worn under the clothes. The absorbent product may also be a bed protection, a seat protection or the like, adapted to protect a mattress or a chair from moisture.

The backsheet, onto which the fastening means is applied, is defined as the side of the product, which faces away from the user during use, i.e. facing towards the garments in case the absorbent product is to be worn under the clothes, and facing towards the mattress in case the product is a bed protection. In any case the backsheet is opposite to a body facing side of the absorbent product.

The method comprises applying an adhesive layer on the backsheet of the absorbent product, and applying an expandable ink, wherein 5-95% of the backsheet is covered by the expandable ink. The adhesive may be a pressure sensitive hot melt adhesive. An advantage of using a pressure sensitive hot melt adhesive is that this type of adhesive is open for adhesion to infinity. The adhesive may be applied to the backsheet at a temperature of 100-180 °C.

The adhesive may preferably be disposed on the backsheet in an even layer, or may be applied in a pattern on the backsheet. The adhesive may be applied by the slot technique, wherein narrow or broad stretches of adhesive may be applied. The adhesive may also be sprayed wherein the width of the adhesive depends on the spray nozzle. Less amount of adhesive may be used when using the spray technique compared with the slot technique.

The expandable ink may be expanded so as to be transformed into three-dimensional protrusions as a result of activation, which will be described in more detail below. Such protrusions obtained after expansion of the ink will be relatively soft and compressible, and may thus be excellent as distances for providing a delayed attachment.

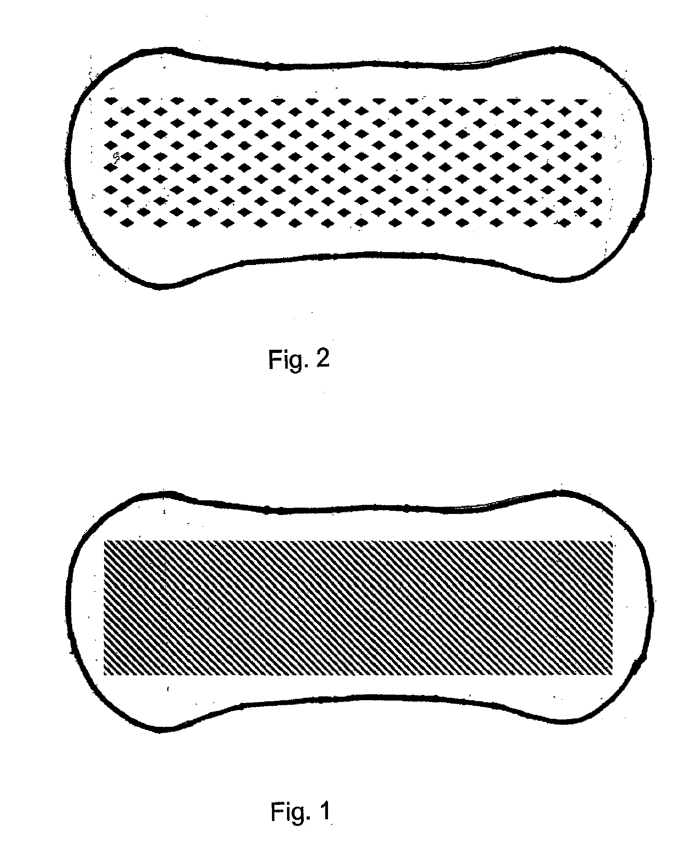

The expandable ink may be a standard formulation known to the skilled man in the art, comprising a polymer, a solvent, binders, thickeners, pigments and a blowing agent or expandable microspheres comprising a propellant. The ink is applied by printing, e.g. by screen printing, flexoprint, ink jet printing, rotary screen printing or gravure. Application by flexoprint is an advantageous printing method, due to its stability, and insensitivity to dust or particles in the ink. The ink may be applied in selected areas as desired, and in any desired pattern. By printing, any desired pattern of protrusions can easily be obtained. For example, expandable ink may be printed in a pattern of diagonal lines on an adhesive surface coating, so that the adhesive is exposed in diagonal narrow spaces between each printed ink line, as shown in Figure 1. Figure 2 shows another example of a printing pattern, where diamond shaped pattern elements are printed on an adhesive surface coating, leaving a grid of intersecting adhesive lines there between. Alternatively, the intersecting grid lines may instead be printed with expandable ink, exposing diamond shaped adhesive areas there between. It is to be understood that various other printing patterns may also be suitable for use in the present invention.

The amount of expandable ink applied to the backsheet of the absorbent product will depend on the composition of the ink, on the desired pattern or on the desired height of the protrusions after activation of the ink. The amount of ink needed may vary also depending on the surface absorbency. The skilled person can find out the amount required to achieve the desired surface by routine experiments.

After application of the ink on the absorbent product, any solvents will evaporate so that the ink dries almost instantaneously.

As said above, the expandable ink may be activated, in order to obtain a desired effect. During activation, components of the ink swell up due to the action of blowing agent or microspheres, so that the applied ink is transformed into three-dimensional structures, which protrude from the surface of the backsheet of the absorbent product. The activation may be performed as a step in the method of applying the fastening means to the absorbent product, thus effectively obtaining a fastening means ready for use on the absorbent product.

The activation may be carried out by exposing the expandable ink to heat, preferably by means of infra-red radiation, hot air, or microwaves. These heating methods allow flexible and versatile heating, which will provide for effective activation of the expandable ink. Alternatively, the ink may be brought into contact with a heated surface, such as an embossing roll. The activation temperature depends on the amount of ink applied and of the type of ink used, and is typically 80 till 180 °C, preferably 100-180 °C.

When the adhesive is a hot melt adhesive, which is applied at a temperature on or above the activation temperature of the expandable ink, the ink can advantageously be applied onto the hot melt adhesive while it is still on or above the activation temperature. For example, if the hot melt adhesive is applied at a temperature of 100-180 °C, the expandable ink may be applied while the hot melt adhesive is still at temperature above 80°C. Thereby, the ink is printed and activated in one step, using the heat of the hot melt adhesive for activation. This increases the energy efficiency and speed of the process, since no equipment for supplying heat to the expandable ink is required.

If the expandable ink is of a type that can be activated by UV radiation, the activation may instead be performed by means of UV radiation, which may be advantageous in particular when heat-sensitive materials are used in the absorbent product.

When it is desired to obtain a pattern of protruding areas, the expandable ink can be activated so as to obtain expansion only in selected areas, while leaving other areas unexpanded. This can be done by masking areas which are not to be activated, or by directing heat or UV radiation only to areas which are to be activated, e.g. by using a directed beam. Hot embossing can also be used. By activation the ink in selected areas only, creation of different protruding patterns on the backsheet of absorbent products having the ink applied in the same pattern can be accomplished.

It may be advantageous to perform the activation of the expandable ink separately, for example when it is desirable to provide absorbent products having all-round fastening means, which can be adapted for attachment to different kinds of surfaces at a later stage. The means of activation as described above for the in-line activation may of course be used also when activating at a later stage.

After activation, the three-dimensional protrusions may be soft and compressible, so that they may act as distances, but can also be compressed, if needed, to allow the adhesive areas to come into contact with the surface to which the absorbent product is to be attached.

The method of the invention may be performed in an apparatus where equipment for carrying out the steps of application of adhesive and expandable ink; activation of the ink (if included in the process) is arranged such that absorbent product blanks can be fed through each step in a line production, e.g. by arranging discrete absorbent product blanks on a conveyor belt, passing each step of the method.

The present invention also pertains to an absorbent product having a fastening means which can be obtained by the above method. The absorbent product may comprise a body facing side of a nonwoven, a film or a laminate thereof and a backsheet of a liquid impervious polymeric film material and an absorbent layer comprising pulp and/or superabsorbent material and/or a fibrous web. The backsheet material may be breathable or non-breathable. The backsheet is facing away from the user during use, and is opposite to the body facing side of the absorbent product. A fastening means applied on the backsheet comprises an adhesive layer and an expandable ink.

When ready for use the expandable ink has been activated so as to form three-dimensionally extending protrusions, having characteristics as described above. The three-dimensionally extending protrusions preferably have a height of 0.1-8 mm, in order to obtain sufficient frictional adhesion against a surface. When the absorbent product is a sanitary napkin or a panty liner, the height of the protrusions may be 0.1-3 mm. A bed sheet may have higher protrusions, up to 8 mm, as the pressing force applied on a bed sheet by a user is higher, due to the weight of the user.

In the fastening means 5-95% of the surface area of the backsheet is covered by the expandable ink which may leave areas of adhesive exposed, thereby allowing initial adjustment of the absorbent product on the surface onto which it is to be attached, and ensuring final attachment to the surface by the adhesive. Preferably, 10-60% of the backsheet may be covered by expandable ink, and 40-90% of the backsheet may be covered by adhesive.

Accordingly, sufficient adhesive is exposed so as to ensure adhesion of the absorbent product to the surface onto which it is to be attached. At the same time, the ink is applied to the backsheet in an amount to initially create enough distance between the surface and the backsheet to allow adjustment of the position of the absorbent product on the surface. Also, the ink when expanded may eliminate the need of release sheets on the absorbent product prior to use.

The invention also pertains to the use of an expandable ink in combination with an adhesive layer as a fastening means for an absorbent product. All details and steps described above in relation to the method and absorbent product are relevant also in relation to this use.

## Claims

1. A method of applying a fastening means to a backsheet of an absorbent product, said backsheet facing away from the user during use, and being opposite to a body facing side of the absorbent product, wherein said method comprises the step of applying an adhesive layer on the backsheet of the absorbent product,
**characterized in that** it further comprises the step of applying by printing an expandable ink, which can be activated by heat or UV radiation, on said adhesive layer, wherein 5-95 % of the backsheet is covered by the expandable ink.

2. The method of claim 1, wherein 10-60 % of the backsheet is covered by the expandable ink.

3. The method of claims 1 or 2, further comprising a step of activating the expandable ink.

4. The method of claim 3, wherein said step of activating is performed by exposing the expandable ink to heat.

5. The method of any of the previous claims, wherein said adhesive is a pressure sensitive hot melt adhesive.

6. The method of claim 4-5, wherein the pressure sensitive hot melt adhesive is applied at a temperature of 100-180 °C, and the expandable ink is applied while the hot melt adhesive is still at a temperature above 80 °C

7. The method of claim 4, wherein the expandable ink is activated by means of infra-red radiation, hot air, microwaves.

8. The method of claim 3, wherein expandable ink is activated by means of UV radiation.

9. The method of claim 3-8, wherein the expandable ink is activated only in selected areas.

10. The method of any of the previous claims, wherein the expandable ink is applied in selected areas.

11. The method of any of the previous claims, wherein the expandable ink comprises a polymer, a solvent and a blowing agent or expandable microspheres.

12. An absorbent product comprising a body facing side, a backsheet facing away from the user during use, and being opposite to the body facing side of the absorbent product, and a fastening means applied on the backsheet, **characterized in that** the fastening means comprises an adhesive layer and a printed expandable ink which can be activated by heat or UV radiation.

13. The absorbent product of claim 12, wherein the expandable ink has been activated so as to form three-dimensionally extending protrusions.

14. The absorbent product of claim 13, wherein the three-dimensionally extending protrusions have a height of 0.1 -8 mm, preferably 0.1 -3 mm.

15. The absorbent product of any one of claims 12-14, wherein 5-95 %, preferably 10-60 % of the backsheet is covered by the expandable ink.

## Patentansprüche

1. Verfahren zum Aufbringen eines Befestigungsmittels auf einer Rückseite eines absorbierenden Produkts, wobei die Rückseite während der Benutzung von dem Benutzer weg zeigt und einer einem Körper zugewandten Seite des absorbierenden Produkts gegenüberliegt, wobei das Verfahren den Schritt des Aufbringens einer Klebelage auf die Rückseite des absorbierenden Produkts umfasst,
**dadurch gekennzeichnet, dass** es ferner den Schritt des Aufbringens durch Drucken einer expandierbaren Tinte, welche durch Wärme oder UV-Strahlung aktiviert werden kann, auf die Klebelage umfasst, wobei 5-95 % der Rückseite von der expandierbaren Tinte bedeckt sind.

2. Verfahren nach Anspruch 1, wobei 10-60 % der Rückseite von der expandierbaren Tinte bedeckt sind.

3. Verfahren nach Anspruch 1 oder 2, ferner umfassend einen Schritt des Aktivierens der expandierbaren Tinte.

4. Verfahren nach Anspruch 3, wobei der Schritt des Aktivierens durch Aussetzen der expandierbaren Tinte gegenüber Wärme durchgeführt wird.

5. Verfahren nach einem der vorhergehenden Ansprüche, wobei der Klebstoff ein druckempfindlicher Hot-melt Klebstoff ist.

6. Verfahren nach Anspruch 4-5, wobei der druckempfindliche Hot-melt Klebstoff bei einer Temperatur von 100-180°C aufgetragen wird und die expandierbare Tinte aufgebracht wird, während der Hot-melt Klebstoff noch bei einer Temperatur oberhalb von 80°C ist.

7. Verfahren nach Anspruch 4, wobei die expandierbare Tinte durch Infrarotbestrahlung, Heißluft, Mikrowellen aktiviert wird.

8. Verfahren nach Anspruch 3, wobei expandierbare Tinte durch UV-Strahlung aktiviert wird.

9. Verfahren nach Anspruch 3-8, wobei die expandierbare Tinte nur in ausgewählten Bereichen aktiviert wird.

10. Verfahren nach einem der vorhergehenden Ansprüche, wobei die expandierbare Tinte in ausgewählten Bereichen aufgebracht wird.

11. Verfahren nach einem der vorhergehenden Ansprüche, wobei die expandierbare Tinte ein Polymer, ein Lösungsmittel und ein Treibmittel oder expandierbare Mikrokugeln umfasst.

12. Absorbierendes Produkt umfassend eine einem Körper zugewandte Seite, eine Rückseite, die während der Benutzung von dem Benutzer weg gerichtet ist und der dem Körper zugewandten Seite des absorbierenden Produkts gegenüberliegt, und ein Befestigungsmittel, das auf der Rückseite aufgebracht ist, **dadurch gekennzeichnet, dass** das Befestigungsmittel eine Klebelage und eine gedruckte expandierbare Tinte umfasst, die durch Wärme oder UV-Strahlung aktiviert werden kann.

13. Absorbierendes Produkt nach Anspruch 12, wobei die expandierbare Tinte aktiviert wurde, um sich dreidimensional erstreckende Vorsprünge zu bilden.

14. Absorbierendes Produkt nach Anspruch 13, wobei die sich dreidimensional erstreckenden Vorsprünge eine Höhe von 0,1-8 mm, bevorzugt 0,1-3 mm aufweisen.

15. Absorbierendes Produkt nach einem der Ansprüche 12-14, wobei 5-95 %, bevorzugt 10-60 % der Rückseite von der expandierbaren Tinte bedeckt sind.

## Revendications

1. Procédé d'application d'un moyen de fixation sur une feuille postérieure d'un produit absorbant, ladite feuille postérieure étant orientée à l'opposé de l'utilisateur en cours d'utilisation, et étant opposée à un côté orienté vers le corps du produit absorbant, ledit procédé comprenant l'étape qui consiste à appliquer une couche d'adhésif sur la feuille postérieure du produit absorbant,
**caractérisé en ce qu'**il comprend en outre l'étape qui consiste à appliquer par impression une encre expansible, qui peut être activée par la chaleur ou par un rayonnement UV, sur ladite couche d'adhésif, 5 à 95 % de la feuille postérieure étant recouverts par l'encre expansible.

2. Procédé selon la revendication 1, dans lequel 10 à 60 % de la feuille postérieure sont recouverts par l'encre expansible.

3. Procédé selon les revendications 1 ou 2, comprenant en outre une étape qui consiste à activer l'encre expansible.

4. Procédé selon la revendication 3, dans lequel ladite étape d'activation est réalisée par exposition de l'encre expansible à la chaleur.

5. Procédé selon l'une quelconque des revendications précédentes, dans lequel ledit adhésif est un adhésif thermofusible sensible à la pression.

6. Procédé selon la revendication 4 ou 5, dans lequel l'adhésif thermofusible sensible à la pression est appliqué à une température de 100 à 180 °C, et l'encre expansible est appliquée tandis que l'adhésif thermofusible se trouve encore à une température supérieure à 80 °C.

7. Procédé selon la revendication 4, dans lequel l'encre expansible est activée au moyen d'un rayonnement infrarouge, d'air chaud, ou de micro-ondes.

8. Procédé selon la revendication 3, dans lequel l'encre expansible est activée au moyen d'un rayonnement UV.

9. Procédé selon les revendications 3 à 8, dans lequel l'encre expansible est activée seulement dans des zones sélectionnées.

10. Procédé selon l'une quelconque des revendications précédentes, dans lequel l'encre expansible est appliquée dans des zones sélectionnées.

11. Procédé selon l'une quelconque des revendications précédentes, dans lequel l'encre expansible comprend un polymère, un solvant et un agent gonflant ou des microsphères expansibles.

12. Produit absorbant comprenant un côté orienté vers le corps, une feuille postérieure orientée à l'opposé de l'utilisateur en cours d'utilisation, et étant opposée au côté orienté vers le corps du produit absorbant, et un moyen de fixation appliqué sur la feuille postérieure, **caractérisé en ce que** le moyen de fixation comprend une couche d'adhésif et une encre expansible imprimée qui peut être activée par la chaleur ou par un rayonnement UV.

13. Produit absorbant selon la revendication 12, dans lequel l'encre expansible a été activée de façon à former des parties saillantes qui s'étendent en trois dimensions.

14. Produit absorbant selon la revendication 13, dans lequel les saillies qui s'étendent en trois dimensions ont une hauteur de 0,1 à 8 millimètres, préférablement de 0,1 à 3 millimètres.

15. Produit absorbant selon l'une quelconque des revendications 12 à 14, dans lequel 5 à 95 %, préférablement 10 à 60 % de la feuille postérieure sont recouverts par l'encre expansible.
